# EUROPEAN PATENT APPLICATION

(11) **EP 2 985 050 A1**
(43) Date of publication of application: **17.02.2016**
(21) Application number: 14180666.1
(22) Date of filing: 12.08.2014
(51) Int. Cl.: A61M 16/00, A61M 16/12, A61M 16/10, A61M 16/08, A61M 16/20

(54) **Method of upgrading clinical oxygen supply arrangement**

(71) Applicant: Linde Aktiengesellschaft, 80331 München (DE)
(72) Inventor: Dr. Häussermann, Sabine, 82061 Neuried (DE)
(74) Representative: m patent group

(57) **Abstract**

A method of upgrading a clinical oxygen supply arrangement (100) adapted to supply supplemental oxygen to a patient, comprising an oxygen wall outlet (1), a manually operable oxygen flow setting device (2) coupled to the oxygen wall outlet (1), and an oxygen supply line (3) coupled to the oxygen flow setting device (2) and adapted to provide oxygen to a patient (10) in need of oxygen supply via an oxygen delivery device (6). The method is characterized by providing an oxygen saturation sensing device (7) attachable to the patient (10) and adapted to provide data indicative of an oxygen saturation of the blood of the patient (10) on the basis of a noninvasive measurement thereof, and by substituting the manually operable oxygen flow setting device (2) by an automatically operable oxygen flow setting device (5) comprising control means (51) adapted to control an oxygen flow through the automatically operable oxygen flow setting device (5) setting device on the basis of the data from the oxygen saturation sensing device (7) in an automatic operation mode of the automatically operable oxygen flow setting device (5). A clinical oxygen supply arrangement (100) is also subject of the invention.

## Description

The invention relates to a method of upgrading a clinical oxygen supply arrangement adapted to supply supplemental oxygen to a patient and to a corresponding clinical oxygen supply arrangement according to the independent claims.

### Prior art

Oxygen supply arrangements for clinical purposes may comprise an oxygen wall outlet to which a manually operable oxygen flow setting device is coupled. The manually operable oxygen flow setting device is typically equipped with a so called rotameter, i.e. with a device measuring the flow rate of oxygen gas in a closed tube as originally described in DE 215 225 A, or with another flow rate indicator.

While, for extensive surgical treatment, a patient in need of oxygen supply is typically intubated and exclusively supplied with oxygen by means of a respiratory unit, also termed "ventilator", this is typically not the case in emergency rooms, during recovery of acute respiratory patients and during some forms of critical care treatment where patients are not completely ventilated but still need supplemental oxygen. In the latter cases, patients are typically supplied with the supplemental oxygen directly from the wall outlet under control of the manually operable oxygen flow setting device described above. If at all, only comparatively simple respiratory units are used in such cases, e.g. devices for providing so called bilevel positive airway pressure (BiPAP) or continuous positive airway pressure (CPAP). However, under supplemental oxygen supply without sophisticated ventilators also (smaller) surgical interventions may be performed, especially under local anesthetics.

If supplemental oxygen is supplied to a patient, the oxygen saturation of the blood of the patient should measured. Especially in cases of supplemental oxygen supply over longer periods of time, oxygen is conventionally measured intermittently. Oxygen measurement is typically performed non-invasively via a so called oximeter. In oximeters, typically a sensor is placed on a thin and therefore optically translucent part of the patient's body, usually a fingertip or an earlobe, and light of two wavelengths is passed through this body part to a photodetector. The absorbance of each of the two wavelengths is measured, allowing a determination of the absorbance of the pulsing arterial blood alone without interference of peripheral blood.

On the basis of an indication of an oxygen saturation sensing device, a nurse or other personnel responsible for the oxygen supply manually regulates the oxygen flow to the patient by turning a valve of the manually operable oxygen flow setting device.

From US 6,142,149 A, a mobile system providing an automatically regulated oxygen supply to a patient is known, comprising an automatically operable oxygen flow setting device carried by the patient. US 5,365,922 A, US 6,470,885 A and US 6,532,958 A also disclose automatically operable oxygen flow setting devices.

While automatically operable oxygen flow setting devices are known from the prior art, they have never found acceptance in the clinical contexts described above, presumably due to the high effort for establishing completely new oxygen supply arrangements and due to the necessity of changing long-term habits of clinical personnel.

It is therefore the object of the present invention to provide improved means of especially supplemental clinical oxygen supply including automatically operable oxygen flow setting devices.

### Summary of the invention

According to the invention, a method of upgrading a clinical oxygen supply arrangement adapted to supply supplemental oxygen to a patient and a corresponding clinical oxygen supply arrangement according to the independent claims is provided. Preferred embodiments of the invention are subject of the dependent claims and of the description that follows.

As mentioned before, the present invention is used in oxygen supply arrangements wherein a patient is supplied with supplemental oxygen directly from an oxygen wall outlet via an oxygen flow setting device, e.g. by use of a laryngeal mask or a nasal cannula, but without the use of sophisticated ventilators that completely substitute autonomous breathing. The invention thus relates to cases wherein an oxygen flow to the patient is directly influencable via regulation of the oxygen flow setting device, while in ventilators the ventilator itself regulates the oxygen flow.

The current invention is based on the finding that an exchange of only the existing manually operable oxygen flow setting devices as described above to automatically operable oxygen flow setting devices that are specifically adapted to be exchangeable therewith, leaving the rest of the clinical oxygen supply arrangement untouched, will provide broader acceptance of such improved clinical oxygen supply arrangements and, consequently, allow for a better oxygen supply to patients which is less prone to human error. Conventionally, in the entirely manual method as described above, an operator of a manually operable oxygen flow setting device may, e.g. due to stress during the treatment of emergency cases or surgical treatment, omit to up regulate the oxygen flow or, in some cases with even more severe consequences, forget to down regulate a previously up regulated oxygen flow, thus providing excessive oxygen amounts to the patient.

Conventionally, during the treatment of emergency cases or surgical treatment, the paradigm "as much oxygen as possible" was followed. More modern methods of treatment, however, suggest supplying "titrated oxygen", i.e. provide an optimum oxygen saturation, especially in patient groups which are extremely sensitive to excessive oxygen supply, e.g. patients with chronical obstructive lung disease (COPD). In such patients, excessive oxygen supply might result in reduced breathing or the complete stop of breathing. In case of myocardial infarction, seizures or stroke, a high peripheral blood perfusion is needed. Excessive oxygen supply, however, results in the reduction of peripheral blood flow and is therefore contraindicated.

Especially in these modern methods of treatment with tight oxygen titration, a manual operation of entirely manually operable oxygen flow setting devices becomes difficult or even impossible in the hectic environment of a hospital, since it would take time. It is highly likely that an operator, in an entirely manually operation, leaves the optimum range of the oxygen supply for specific types of patients. Completely exchanging existing clinical supply arrangements for establishing improved and automatically controllable systems would however include, as mentioned, high investion costs and/or a change of long-established habits in clinical practice.

### Advantages of the invention

According to the current invention, a method of upgrading a clinical oxygen supply arrangement adapted to supply supplemental oxygen to a patient, comprising an oxygen wall outlet, a manually operable oxygen flow setting device coupled to the oxygen wall outlet, and an oxygen supply line coupled to the manually operable oxygen flow setting device and adapted to provide oxygen to a patient in need of oxygen supply via an oxygen delivery device, especially, as mentioned, via a laryngeal mask and/or a nasal cannula, is provided.

According to the invention, to upgrade such a clinical oxygen supply arrangement, it is suggested to provide an oxygen saturation sensing device attachable to the patient and adapted to provide data indicative of an oxygen saturation of the blood of the patient on the basis of a non-invasive measurement thereof. Furthermore, it is suggested to substitute the manually operable oxygen flow setting device by an automatically operable oxygen flow setting device comprising control means adapted to control an oxygen flow through the automatically operable oxygen flow setting device on the basis of the data from the oxygen saturation setting device in an automatic operation mode of the automatically operable oxygen flow setting device.

In other words, the current invention suggests leaving the existing clinical oxygen supply arrangement largely untouched and exchanging only the existent manually operable oxygen flow setting device by an oxygen flow setting device which is, at least in an automatic operation mode, entirely automatically operable.

Most advantageously, the substitution of the manually operable oxygen flow setting device by the automatically operable oxygen flow setting device simply comprises uncoupling of the manually operable oxygen flow setting device from and coupling of the automatically operable oxygen flow setting device to the existing oxygen wall outlet and the existing oxygen supply line. In its simplest form, the invention is thus realized by simply mounting the automatically operable oxygen flow setting device in between the wall outlet and the oxygen supply line, substituting the previously present manually operable oxygen flow setting device, especially a rotameter with a corresponding manually operable valve. An automatically operable oxygen flow setting device which is usable according to the present invention is therefore specifically adapted to be mutually exchangeable to existing manually operable oxygen flow setting devices and may include suitable coupling means, optionally including adapters to adapt to different tubing and/or coupling systems of oxygen supply lines and/or oxygen wall outlets. A corresponding coupling may be realized e.g. by screwing, push-fitting and/or pressfitting of the corresponding lines and ports.

Advantageously, the oxygen flow setting device used in the method according to the present invention comprises communication means adapted to communicate at least with the oxygen saturation sensing device. Under a "communication", according to the current application, any means of data transfer between two units is to be understood. Communication especially includes mono- and bidirectional data exchange. This means, also simply sending of data indicative of an oxygen saturation of the blood of the patient from the oxygen saturation sensing device attached to the patient to the inventively provided automatically operable oxygen flow setting device is a form of communication. A communication, according to the present invention, may be realized by different communication protocols, media and/or layers, e.g. via Bluetooth, USB connections, Ethernet, conventional serial links and the like.

Most preferably, the communication means of the automatically operable oxygen flow setting device provided according to the present invention comprise at least one receiver which is adapted to wiredly and/or wirelessly receive at least the data from the oxygen saturation sensing device which are indicative of the oxygen saturation of the blood of the patient. At least one receiver of the communication means may also be adapted to receive data from other units provided in a clinical setup, e.g. handheld devices adapted to input data, especially patient data nor treatment instructions, other oxygen supply arrangements and/or monitoring devices, as also explained below.

Correspondingly, the communication means of the automatically operable oxygen flow setting device used according to the present invention may also comprise at least one sender which is adapted to wiredly and/or wirelessly send data to at least a monitoring unit and/or at least one other unit provided in the clinical setup. A monitoring unit provided according to a preferred embodiment of the present invention may be adapted to monitor the status of a number of automatically operable oxygen flow setting devices provided in a number of clinical oxygen supply arrangements which are the result of an upgrading method according to the present invention.

Such a monitoring unit may be especially used to detect any malfunction and/or unexpected behavior of an automatically operable oxygen flow setting device and/or unexpected events relating to a status of one or more patients. Correspondingly, a preferred embodiment of the present invention includes that the data the at least one sender is adapted to send is indicative of a status of the patient and/or of the automatically operable oxygen flow setting device. Providing a correspondingly adapted sender, it is possible for the clinical personnel to immediately react, e.g. in unexpected and/or emergency situations.

In this context, it is preferred that the automatically operable oxygen flow setting device is further adapted to also be operated manually in at least one manual operation mode. A manual operation mode may for example be chosen if an unexpected status of the patient and/or of the automatically operable oxygen flow setting device is registered by the monitoring unit described above. The monitoring unit as described above may in this case also be adapted to monitor a signal continuously sent by the automatically operable oxygen flow setting device. If such a signal, corresponding to e.g. a known dead-man's signal, is absent, the monitoring device may deduce that the automatically operable flow setting device is unresponding and/or malfunctioning. In such cases, the monitoring device may issue a signal indicative of a malfunction of the automatically operable oxygen flow setting device and instruct the personnel to perform a manual operation of the automatically operable oxygen flow setting device.

In other words, it is preferred that the at least one manual operation mode is initiated if a determination is made that the automatically operable oxygen flow setting device is malfunctioning and/or that the status of the patient is an unexpected one. Consequently, a non-responsiveness and/or malfunctioning of the automatically operable oxygen flow setting device may be early detected and an immediate action may be taken, minimizing the risk of harm to the patient.

As explained above, the determination that the automatically operable oxygen flow setting device is malfunctioning is preferentially made on the basis of data sent by the automatically operable flow setting device. Especially, if such data are implausible and/or cease to be sent, a corresponding determination may be made.

According to a preferred embodiment of the present invention, the automatically operable oxygen flow setting device comprises a memory unit adapted to store patient and/or treatment data relating to the patient and/or a history of data provided by the oxygen saturation setting device. A corresponding memory unit may especially comprise a fixed or detachable memory device, e.g. fixed flash memory and/or a removable flash memory card or an USB key which may be inserted in a corresponding memory unit. On a corresponding memory device of the memory unit, the mentioned data may be stored, especially in encrypted form to meet privacy protection prerequisites. In such a memory unit and/or a corresponding memory device, the whole treatment history of the treatment of one or more patients may be stored and provided for later documentation. Possible treatment errors or unexpected events in the treatment may in this way be identified and provided as a basis for further treatment. It is also possible to send corresponding data to an external device, especially via the previously mentioned communication unit, to store such data in the external device for documentation and/or any other further use.

The automatically operable oxygen flow setting device provided according to the present invention may especially include a proportional-integral-derivative (PID) controller, as generally known in the field of oxygen supply.

The automatically operable oxygen flow setting device provided according to the present invention may especially be adapted to store treatment data relating to the patient, e.g. including a specific oxygen demand of the patient to avoid excessive oxygen supply, as mentioned above.

According to a further preferred embodiment of the invention, the automatically operable oxygen flow setting device may be adapted to correlate the patient and/or treatment data mentioned above with the data provided by the oxygen saturation sensing device indicative of the oxygen saturation of the blood of the patient. If e.g. a discrepancy between the latter and the patient and/or treatment data is identified and the discrepancy exceeds a predefined threshold for more than a predetermined time, a signal, as explained above, to a monitoring unit may be issued. In this case, the monitoring unit may initiate a manual operation of the automatically operable oxygen flow setting device, leaving the ultimate decision on how to proceed to human skill.

A clinical oxygen supply arrangement adapted to supply supplemental oxygen to a patient, comprising an oxygen wall outlet, an oxygen flow setting device coupled to the oxygen wall outlet, and an oxygen supply line coupled to the oxygen flow setting device and adapted to provide oxygen to a patient in need of oxygen supply via an oxygen delivery device is also subject of the present invention.

According to the present invention, the clinical oxygen supply arrangement comprises an oxygen saturation setting device attachable to the patient and adapted to provide data indicative of an oxygen saturation of the blood of the patient on the basis of a non-invasive measurement thereof, and the oxygen flow setting device is automatically operable and comprises control means adapted to control an oxygen flow through the automatically operable oxygen flow setting device on the basis of the data from the oxygen saturation sensing device in an automatic operation mode of the automatic operable oxygen flow setting device. Furthermore, according to the present invention, the automatically operable oxygen flow setting device is mutually exchangeable to a manually operable oxygen flow setting device as known in the prior art. Especially, the clinical oxygen supply arrangement is provided by a method as previously explained.

The invention and further aspects of the invention are also described on the basis of the appended drawing.

### Short description of the drawing

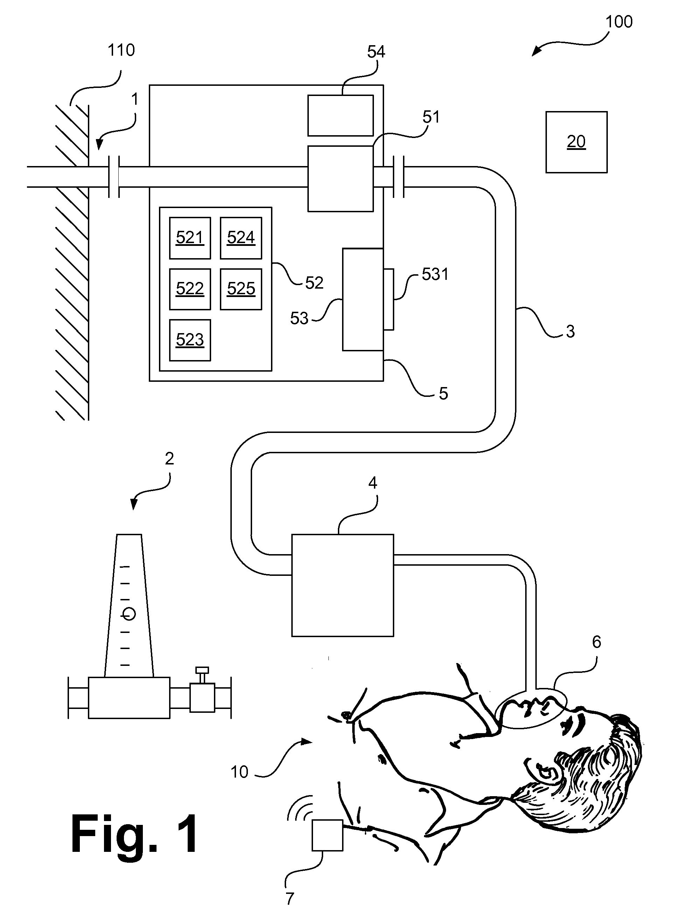

Figure 1 shows a clinical oxygen supply arrangement according to a preferred embodiment of the invention.

### Embodiment of the invention

In Figure 1, a clinical oxygen supply arrangement adapted to supply supplemental oxygen to a patient according to a preferred embodiment of the invention is shown and indicated with 100. The clinical oxygen supply arrangement 100 comprises an oxygen wall outlet 1 in a wall 110 of an emergency room, a critical care room or a pneumology or general ward to which an automatically operable oxygen flow setting device 5 is directly coupled. According to the present invention, the automatically operable oxygen flow setting device 5 is provided in substitute to a manually operable oxygen flow setting device 2 and mutually exchangeable therewith.

An oxygen supply line 3 is coupled to the automatically operable oxygen flow setting device. Coupling between the oxygen wall outlet 1 and the automatically operable oxygen flow setting device 5 and between the automatically operable oxygen flow setting device 5 and the oxygen supply line 3 is provided via standard couplings also provided at a manually operable oxygen flow setting device 2 as conventionally present. Via the oxygen supply line 3, a unit 4 is supplied with oxygen, which in turn provides the oxygen and/or a corresponding gas mixture to a patient 10 via an oxygen delivery device 6, e.g. via a suitable mask and/or a nasal cannula (not shown). As mentioned, the unit 4 is at most embodied as a comparatively simple ventilator, e.g. as a device for providing bilevel positive airway pressure or continuous positive airway pressure. The unit 4 does not represent a sophisticated respiratory unit as used in highly invasive surgery, e.g. for totally substituting autonomous breathing. The invention is especially useful in cases where e.g. a mask is directly coupled with the oxygen supply line 3.

An oxygen saturation sensing device 7 is provided and attached to the patient, for example to a finger of the patient, and is adapted to provide data indicative of an oxygen saturation of the blood of the patient on the basis of a non invasive measurement thereof. The oxygen saturation sensing device is further adapted to communicate, in the sense as explained above, with the automatically operable oxygen flow setting device via a communication channel, for example via a wireless communication channel. The communication may, as mentioned, be unidirectional, i.e. the oxygen flow sensing device 7 may also be adapted to only send corresponding data to the automatically operable oxygen flow setting device 5.

According to an embodiment of the invention, the automatically operable oxygen flow setting device 5 may comprise control means 51 adapted to control an oxygen flow through the automatically operable oxygen flow setting device 5 on the basis of the data from the oxygen saturation sensing device 7 in an automatic operation mode. The control means may comprise automatically operable valves and/or flow measuring devices which are not shown in detail.

Furthermore, the automatically operable oxygen flow setting device 5 may comprise communication means 52 which include at least one receiver 521, 522, 523 and/or at least one sender 524, 525, the at least one receiver 521, 522, 523 being adapted to wiredly and/or wirelessly receive at least the data from the oxygen saturation sensing device 7 and/or the at least one sender 524, 525 being adapted to wiredly and/or wirelessly send data to at least one monitoring unit 20. The automatically operable oxygen flow setting device 5 may also comprise a memory unit 53 including a memory device 531, e.g. a flash card or a fixedly welded flash memory device. The automatically operable oxygen flow setting device 5 may also include a user interface 54 adapted to input data and/or to present data to a user of the clinical oxygen supply arrangement, e.g. a keyboard, a monitor, and/or a touchpad.

## Claims

1. A method of upgrading a clinical oxygen supply arrangement adapted to supply supplemental oxygen to a patient, comprising an oxygen wall outlet (1), a manually operable oxygen flow setting device (2) coupled to the oxygen wall outlet (1), and an oxygen supply line (3) coupled to the manually operable oxygen flow setting device (2) and adapted to provide oxygen to a patient (10) in need of oxygen supply via an oxygen delivery device (6), **characterized by** providing an oxygen saturation sensing device (7) attachable to the patient (10) and adapted to provide data indicative of an oxygen saturation of the blood of the patient (10) on the basis of a non-invasive measurement thereof, and by substituting the manually operable oxygen flow setting device (2) by an automatically operable oxygen flow setting device (5) comprising control means (51) adapted to control an oxygen flow through the automatically operable oxygen flow setting device (5) on the basis of the data from the oxygen saturation sensing device (7) in an automatic operation mode of the automatically operable oxygen flow setting device (5).

2. A method according to claim 1, wherein the substitution of the manually operable oxygen flow setting device (2) by the automatically operable oxygen flow setting device (5) comprises uncoupling of the manually operable oxygen flow setting device (2) from and coupling of the automatically operable oxygen flow setting device (5) to the oxygen wall outlet (1) and the oxygen supply line (3).

3. A method according to claim 1 or 2, wherein the automatically operable oxygen flow setting device (2) comprises communication means (52) adapted to communicate at least with the oxygen saturation sensing device (7).

4. A method according to claim 3, wherein the communication means (52) comprise at least one receiver (521, 522, 523) adapted to wiredly and/or wirelessly receive at least the data from the oxygen saturation sensing device (7).

5. A method according to claim 3 or 4, wherein the communication means (52) comprise at least one sender (524, 525) adapted to wiredly and/or wirelessly send data at least to a monitoring unit (20).

6. A method according to claim 5, wherein the data the at least one sender is adapted to send are indicative of a status of the patient (10) and/or of the automatically operable oxygen flow setting device (2).

7. A method according to any of the preceding claims, wherein the automatically operable oxygen flow setting device (5) is further adapted to be operated manually in at least one manual operation mode.

8. A method according to claim 7, wherein the at least one manual operation mode is initiated if a determination is made that the automatically operable oxygen flow setting device (5) is malfunctioning.

9. A method according to claim 8, wherein the determination that the automatically operable oxygen flow setting device (5) is malfunctioning is made on the basis of data sent by the automatically operable oxygen flow setting device (5).

10. A method according to any of the preceding claims, wherein the automatically operable oxygen flow setting device (5) comprises a memory unit (53) adapted to store patient and/or treatment data relating to the patient (10).

11. A method according to any of the preceding claims, wherein the automatically operable oxygen flow setting device (5) comprises an user interface (54) adapted to provide and/or receive patient data, treatment data and/or data based on the data from the oxygen saturation sensing device (7).

12. A clinical oxygen supply arrangement adapted to supply supplemental oxygen to a patient, comprising an oxygen wall outlet (1), an oxygen flow setting device (5) coupled to the oxygen wall outlet (1), and an oxygen supply line (3) coupled to the oxygen flow setting device (5) and adapted to provide oxygen to a patient (10) in need of oxygen supply via an oxygen delivery device (6), **characterized in that** the clinical oxygen supply arrangement comprises an oxygen saturation sensing device (7) attachable to the patient (10) and adapted to provide data indicative of an oxygen saturation of the blood of the patient (10) on the basis of a noninvasive measurement thereof, **in that** the oxygen flow setting device (5) is automatically operable and comprises control means (51) adapted to control an oxygen flow through the automatically operable oxygen flow setting device (5) on the basis of the data from the oxygen saturation sensing device (7) in an automatic operation mode of the automatically operable oxygen flow setting device (5), and **in that** the automatically operable oxygen flow setting device (5) is mutually exchangeable to a manually operable oxygen flow setting device (2).

13. A clinical oxygen supply arrangement (100) according to claim 12 provided by a method of any one of claims 1 to 11.
